# EUROPÄISCHE PATENTSCHRIFT

(11) **EP 0 848 747 B1**
(45) Veröffentlichungstag und Bekanntmachung des Hinweises auf die Patenterteilung: **15.12.1999**
(21) Anmeldenummer: 96923927.6
(22) Anmeldetag: 26.06.1996
(51) Int. Cl.: C11D 1/835, A61K 7/50

(54) **NIOTENSIDKONZENTRATE**
NONIONIC SURFACTANT CONCENTRATES
CONCENTRES DE TENSIOACTIFS NON IONIQUES

(30) Priorität: 04.07.1995 DE 19524244
(43) Veröffentlichungstag der Anmeldung: 24.06.1998
(73) Patentinhaber: Cognis Deutschland GmbH, 40589 Düsseldorf (DE)
(72) Erfinder: FABRY, Bernd, D-41352 Korschenbroich (DE)
(86) Internationale Anmeldenummer: EP9602776
(87) Internationale Veröffentlichungsnummer: WO9702335

(56) Entgegenhaltungen:
- EP-A- 0 593 406
- WO-A-92/06160
- WO-A-92/22629

## Beschreibung

### Gebiet der Erfindung

Die Erfindung betrifft Niotensidkonzentrate mit einem Gehalt an Fettsäure-N-alkylpolyhydroxyalkylamiden und Alkoholpolyglycolethern ausgewählten HLB-Wertes, ein Verfahren zu ihrer Herstellung sowie die Verwendung einer definierten Mischung von Alkoholpolyglycolethern zur Inhibierung der Kristallbildung in Konzentraten von Fettsäure-N-alkylpolyhydroxyalkylamiden.

### Stand der Technik

Fettsäure-N-alkylglucamide stellen bevorzugte Rohstoffe zur Herstellung von zahlreichen oberflächenaktiven Mitteln wie beispielsweise Haarshampoos oder Handgeschirrspülmittel dar. Aus ökonomischen Gründen ist es bevorzugt, Halbfertigprodukte mit möglichst geringem Wassergehalt herzustellen, um auf diese Weise Lager- und Transportkosten zu minimieren. Bei der Herstellung von wäßrigen Konzentraten dieser Tenside, die über einen nichtwäßrigen Gehalt an Inhaltsstoffen oberhalb von etwa 40 Gew.-% verfügen, treten jedoch eine Reihe von Problemen auf. Werden beispielsweise derartige Zubereitungen bei niedrigen Temperaturen in einem Außenlager aufbewahrt, so findet sehr rasch eine Kristallisation statt, die sich in der Regel als irreversibel erweist. Derartig kristallisierte Produkte können also nur noch verworfen werden. Konzentrate der genannten Art sind in der Regel auch mittel- bis hochviskos, was ihre Pumpbarkeit stark beeinträchtigten kann. Zudem kann es zu unerwünschten Austrübungen und trotz des hohen Tensidgehaltes zu einer Verkeimung kommen, die die Anwesenheit nicht immer erwünschter Konservierungsstoffe erfordert.

Die komplexe Aufgabe der Erfindung hat somit darin bestanden, Niotensidkonzentrate zur Verfügung zu stellen, mit deren Hilfe sich wäßrige Zuckertensidkonzentrate mit einem Gehalt an nichtwäßrigen Inhaltsstoffen ("Aktivgehalt") von mindestens 30 und vorzugsweise 30 bis 100 Gew.-% erhalten lassen, die gleichzeitig niedrigviskos, trübungsfrei und gegenüber Verkeimung weitgehend stabilisiert sind sowie auch bei längerer Lagerung bei niedrigen Temperaturen keine Tendenz zur Kristallisation zeigen.

### Beschreibung der Erfindung

Gegenstand der Erfindung sind Niotensidkonzentrate, enthaltend
a) Fettsäure-N-alkylpolyhydroxyalkylamide und
b) mindestens zwei Alkoholpolyglycolether, von denen mindestens einer einen HLB-Wert unterhalb von 5 und mindestens einer einen HLB-Wert oberhalb von 5 aufweist.

Überraschenderweise wurde gefunden, daß der Zusatz von mindestens zwei Alkoholpolyglycolethern unterschiedlichen HLB-Wertes zur Bildung von eutektischen Gemischen mit einer in synergistischerweise verminderten Kristallisationstemperatur führt. Gleichzeitig sind die Gemische frei von Trübungen, auch in Abwesenheit von Konservierungsmitteln weitgehend gegenüber den Befall durch Mikroorganismen geschützt und niedrigviskos, d.h. leicht pump- und förderbar.

### Fettsäure-N-alkylpolyhydroxyalkylamide

Fettsäure-N-alkylpolyhydroxyalkylamide stellen nichtionische Tenside dar, die der Formel **(I)** folgen, 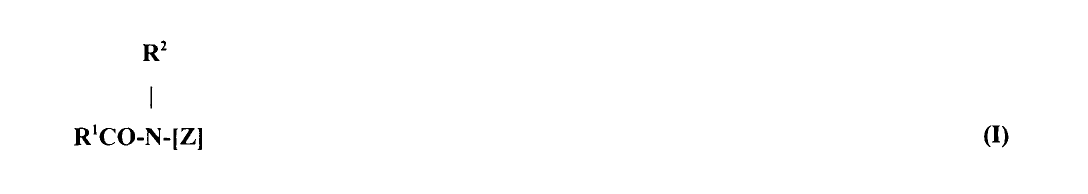 in der R¹CO für einen aliphatischen Acylrest mit 6 bis 22 Kohlenstoffatomen, R² für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf. Det. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(II)** wiedergegeben werden:

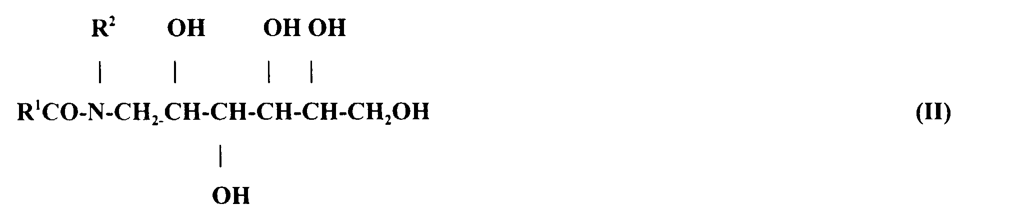

Vorzugsweise werden als Fettsäure-N-alkylpolyhydroxyalkylamide Glucamide der Formel **(II)** eingesetzt, in der R² für eine Alkylgruppe steht und R¹CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel **(II),** die durch reduktive Aminierung von Glucose mit Methylamin und anschließende Acylierung mit Stearinsäure, C_{12/18}-Kokosfettsäure und insbesondere C_{16/18}-Talgfettsäure bzw. einem entsprechenden Derivat erhalten werden. Weiterhin können sich die Polyhydroxyalkylamide auch von Maltose und Palatinose ableiten.

### HLB-Wert

Bei dem von Griffin 1950 erstmals eingeführten HLB-Wert (hydrophilic/lipophilic balance) handelt es sich um ein international etabliertes Maß für die Wasser- bzw. Öllöslichkeit von nichtionischen Tensiden und Emulsionen in der Kosmetik. Eine einfache Methode zur Berechnung des HLB-Wertes nichtionischer Tenside ist der Veröffentlichung von O'Lennick und McCutchen in **Soap Cosm. Chem. Spec. Januar, 34 (1988)** zu entnehmen. Dazu dividiert man den Anteil der hydrophilen Komponente (z.B. eines Alkoholpolyglycolethers) am Gesamtmolekulargewicht durch den Faktor 5. So hat beispielsweise ein Addukt von durchschnittlich 5 Mol Ethylenoxid an Oleylalkohol einen hydrophilen Anteil von 220 am Molekulargewicht 489, entsprechend 45 %. Dividiert durch den Faktor 5, ergibt sich ein HLB-Wert von 9.

### Alkoholpolyglycolether

Alkoholpolyglycolether stellen bekannte nichtionische Tenside dar, die man durch Anlagerung von Ethylenoxid und/oder Propylenoxid an primäre Alkohole erhält. Großtechnisch erfolgt die Alkoxylierung in Gegenwart basischer Katalysatoren, wie beispielsweise Natriummethylat oder calciniertem Hydrotalcit. Dementsprechend können die Alkoholpolyglycolether eine konventionell breite oder aber eine eingeengte Homologenverteilung aufweisen.

Typische Beispiele für Alkoholpolyglycolether mit HLB-Werten unterhalb bzw. oberhalb von 5 stellen die Addukte von Ethylenoxid und/oder Propylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen dar, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind Anlagerungsprodukte von Ethylenoxid an technische Alkohole mit 8 bis 12 Kohlenstoffatomen wie beispielsweise 2-Ethylhexanol, Laurylalkohol, Kokosfettalkohol oder Isotridecylalkohol. Aus anwendungstechnischer Sicht ist der Einsatz von vollständig oder anteilig verzweigten Alkoholethoxylaten wie beispielsweise Addukten von Ethylenoxid an i-Octanol oder Oxoalkoholschnitte eines Kettenlängenbereiches von 9 bis 13 besonders bevorzugt.

Die Auswahl der geeigneten Alkoholpolyglycolether im Hinblick auf ihren HLB-Wert ist für den Fachmann möglich, ohne hierzu erfinderisch tätig zu werden, da die Erfindung nicht nur an zahlreichen Beispielen illustriert wird, sondern sich die Hydrophilie der oben genannten Addukte wie angegeben sehr leicht berechnen läßt. Als Anhaltspunkt mögen die Formeln **(III)** und **(IV)** dienen.

**R**^{**3**}**(CH**_{**2**}**CH**_{**2**}**O)**_{**m**}**H** **(III)**

**R**^{**4**}**(CH**_{**2**}**CH**_{**2**}**O)**_{**n**}**H** **(IV)**

in denen R³ und R⁴ unabhängig voneinander für lineare oder verzweigte Alkylreste mit 6 bis 12 Kohlenstoffatomen, m für Zahlen von 0,5 bis 2 und n für Zahlen von 3 bis 9 stehen. Im ersten Fall werden überwiegend Alkoholpolyglycolether mit einem HLB-Wert unterhalb von 5, im zweiten Fall oberhalb von 5 erhalten.

In einer bevorzugten Ausführungsform der Erfindung, enthalten die Niotensidkonzentrate mindestens zwei Alkoholpolyglycolether, von denen mindestens einer einen HLB-Wert im Bereich von 2 bis 4 und mindestens einer einen HLB-Wert im Bereich von 8 bis 12 aufweist.

### Niotensidkonzentrate

Die erfindungsgemäßen Niotensidkonzentrate können einen nichtwäßrigen Aktivsubstanzgehalt im Bereich von 30 bis 100 Gew.-% aufweisen. Das bedeutet, daß die Erfindung sowohl wasserfreie Produkte als auch solche mit einem Wassergehalt bis 70 Gew.-% umfaßt. Vorzugsweise werden im Sinne der Erfindung wäßrige Konzentrate mit einem Aktivsubstanzgehalt im Bereich von 40 bis 90 und insbesondere 50 bis 60 Gew.-% erhalten.

Der Gehalt der Fettsäure-N-alkylpolyhydroxyalkylamiden an den Konzentraten liegt üblicherweise im Bereich von 25 bis 90 und insbesondere 40 bis 60 Gew.-%. Der Gehalt an Alkoholpolyglycolethern kann in Summe im Bereich von 5 bis 75, vorzugsweise 10 bis 50 und insbesondere 10 bis 40 Gew.-% liegen. Das Gewichtsverhältnis der Alkoholpolyglycolether mit einem HLB-Wert unterhalb bzw. oberhalb von 5 kann im Bereich von 90 : 10 bis 10 : 90 variieren und liegt üblicherweise im Bereich von 75 : 25 bis 25 : 75 und insbesondere 60 : 40 bis 40 : 60.

### Herstellverfahren

Ein weiterer Gegenstand der Erfindung betrifft ein Verfahren zur Herstellung von Niotensidkonzentraten, bei dem man Fettsäure-N-alkylpolyhydroxyalkylamiden mindestens zwei Alkoholpolyglycolether zusetzt, von denen mindestens einer einen HLB-Wert unterhalb von 5 und mindestens einer einen HLB-Wert oberhalb von 5 aufweist.

### Gewerbliche Anwendbarkeit

Die nach dem erfindungsgemäßen Verfahren erhältlichen Niotensidkonzentrate sind auch in hochkonzentriertem, gegebenenfalls auch wasserfreiem Zustand fließfähig und pumpbar, ohne Zusatz von Konservierungsmitteln gegen den Befall von Bakterien und Pilzen weitgehend geschützt. trübungsfrei und kristallisieren auch bei längerer Lagerung bei niedrigen Temperaturen nicht aus. Sie eignen sich zur Herstellung von oberflächenaktiven Mitteln, wie beispielsweise Wasch-, Spül- und Reinigungsmitteln sowie Mitteln zur Haar- und Körperpflege, in denen sie in Mengen von 1 bis 50, vorzusgweise 3 bis 35 Gew.-% - bezogen auf die Mittel - enthalten sein können.

Ein weiterer Gegenstand der Erfindung betrifft ferner die Verwendung von Mischungen von mindestens zwei Alkoholpolyglycolethern, von denen mindestens einer einen HLB-Wert unterhalb von 5 und mindestens einer einen HLB-Wert oberhalb von 5 aufweist, als Kristallisationsinhibitoren zur Herstellung von Niotensidkonzentraten.

### Beispiele

### Beispiele 1 bis 10, Vergleichsbeispiele V1 bis V5

Zur Beurteilung der anwendungstechnischen Eigenschaften wurden die in Tabelle 1 zusammengestellten Rezepturen über einen Zeitraum von 3 Monaten bei 6°C gelagert. Die Kristallbildung und das Erscheinungsbild wurden subjektiv beurteilt, die Viskosität bei 40°C gemäß DIN 53015. Zur Beurteilung der Verkeimungsstabilität wurden die Pasten mit 10⁶ Bakterien und 10⁵ Pilzen (Bakterienmischung: Staphylococcus aureus, Enterococcus faecium, Escherichia coli, Enterobacter aerogenes, Pseudomonas aeruginosa; Pilzmischung: Candida albicans, Aspergillus niger, Penicillium rubrum, Trichoderma viride) versetzt und die Abtötungszeiten (Bakterien/Pilze) bestimmt. Die Ergebnisse sind in Tabelle 2 zusammengefaßt (Prozentangaben als Gew.-%, ggf. Wasser ad 100 Gew.-%).

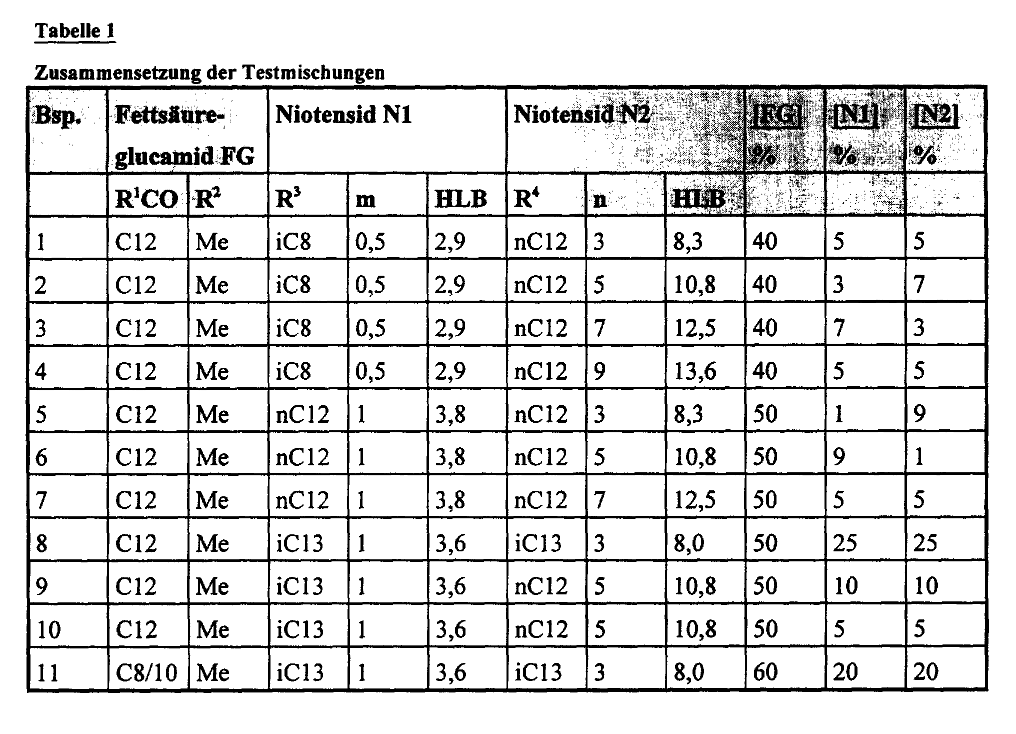

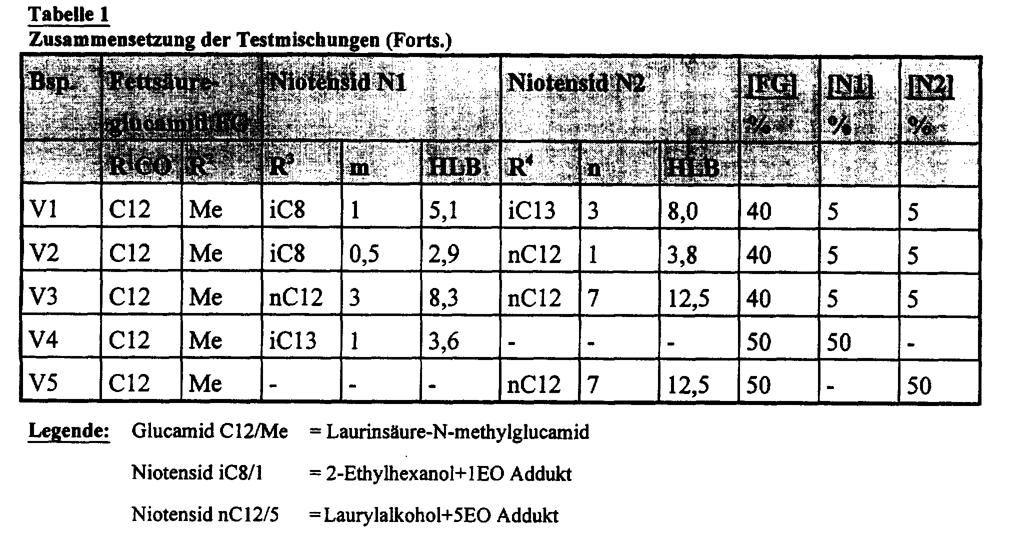

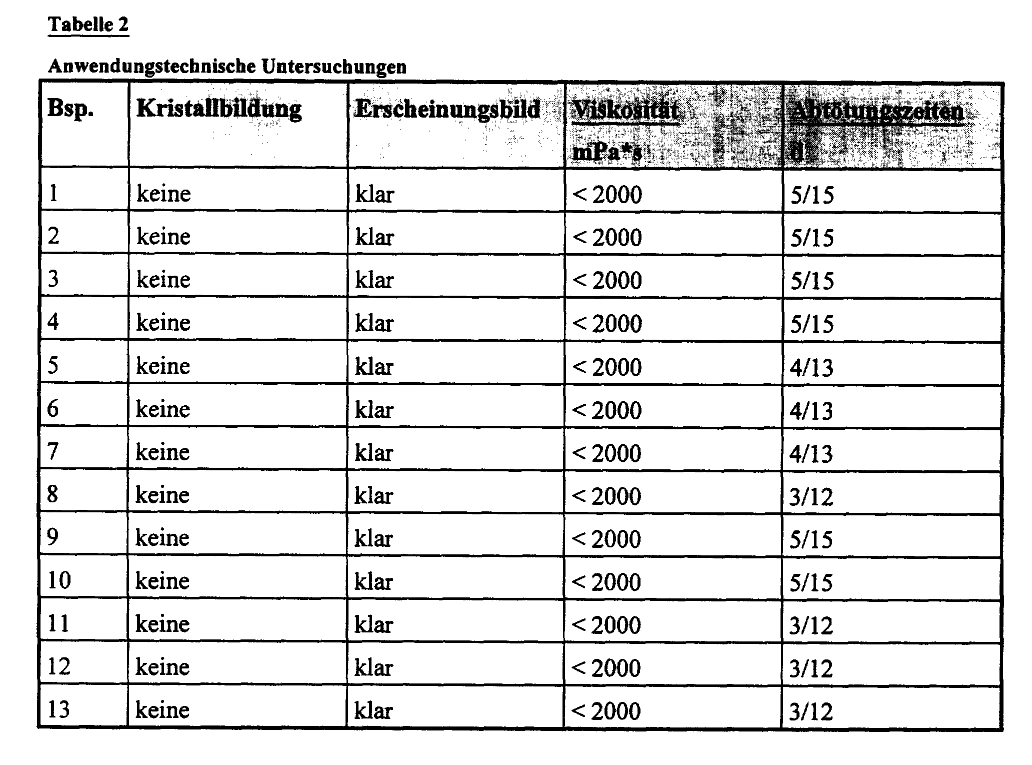

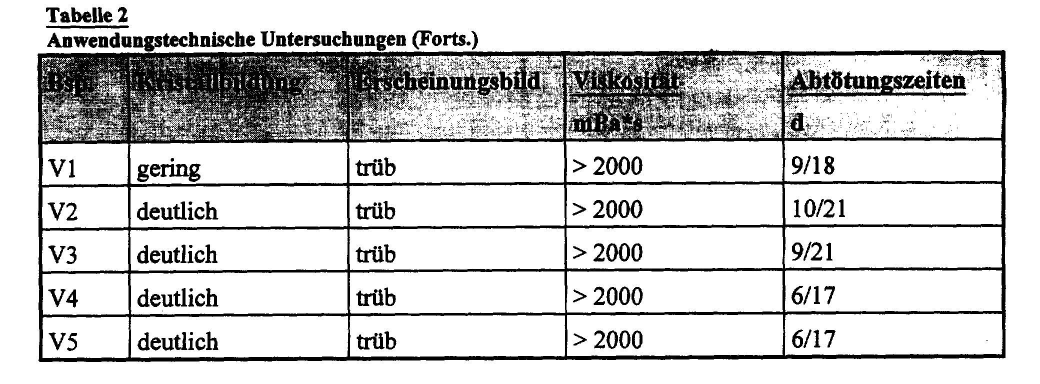

## Patentansprüche

1. Niotensidkonzentrate, enthaltend
a) Fettsäure-N-alkylpolyhydroxyalkylamide und
b) mindestens zwei Alkoholpolyglycolether, von denen mindestens einer einen HLB-Wert unterhalb von 5 und mindestens einer einen HLB-Wert oberhalb von 5 aufweist.

2. Niotensidkonzentrate nach Anspruch 1, **dadurch gekennzeichnet,** daß sie Fettsäure-N-alkyl-polyhydroxyalkylamide der Formel **(I)** enthalten, 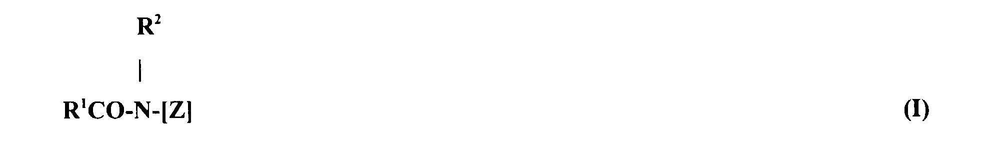 in der R¹CO für einen aliphatischen Acylrest mit 6 bis 22 Kohlenstoffatomen, R² für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen steht..

3. Niotensidkonzentrate nach den Ansprüchen 1 und 2, **dadurch gekennzeichnet,** daß sie Fettsäure-N-alkylglucamide enthalten.

4. Niotensidkonzentrate nach den Ansprüchen 1 bis 3, **dadurch gekennzeichnet,** daß sie mindestens zwei Alkoholpolyglycolether enthalten, von denen mindestens einer einen HLB-Wert im Bereich von 2 bis 4 und mindestens einer einen HLB-Wert im Bereich von 8 bis 12 aufweist.

5. Niotensidkonzentrate nach den Ansprüchen 1 bis 4, **dadurch gekennzeichnet,** daß sie einen Aktivsubstanzgehalt im Bereich von 30 bis 100 Gew.-% aufweisen.

6. Niotensidkonzentrate nach den Ansprüchen 1 bis 5, **dadurch gekennzeichnet,** daß sie einen Gehalt an Fettsäure-N-alkylpolyhydroxyalkylamiden im Bereich von 25 bis 90 Gew.-% aufweisen.

7. Niotensidkonzentrate nach den Ansprüchen 1 bis 6, **dadurch gekennzeichnet,** daß sie einen Gehalt an Alkoholpolyglycolethern im Bereich von 5 bis 75 Gew.-% aufweisen.

8. Niotensidkonzentrate nach den Ansprüchen 1 bis 7, **dadurch gekennzeichnet,** daß sie die Alkoholpolyglycolether mit einem HLB-Wert unterhalb bzw. oberhalb von 5 im Gewichtsverhältnis 10 : 90 bis 90 : 10 enthalten.

9. Verfahren zur Herstellung von Niotensidkonzentraten, bei dem man Fettsäure-N-alkylpolyhydroxyalkylamiden mindestens zwei Alkoholpolyglycolether zusetzt, von denen mindestens einer einen HLB-Wert unterhalb von 5 und mindestens einer einen HLB-Wert oberhalb von 5 aufweist.

10. Verwendung von Mischungen von mindestens zwei Alkoholpolyglycolethern, von denen mindestens einer einen HLB-Wert unterhalb von 5 und mindestens einer einen HLB-Wert oberhalb von 5 aufweist, als Kristallisationsinhibitoren zur Herstellung von Niotensidkonzentraten gemäß Anspruch 1.

## Claims

1. Nonionic surfactant concentrates containing
a) fatty acid-N-alkyl polyhydroxyalkylamides and
b) at least two alcohol polyglycol ethers of which at least one has an HLB value below 5 and at least one an HLB value above 5.

2. Nonionic surfactant concentrates as claimed in claim 1, characterized in that they contain fatty acid-N-alkyl polyhydroxyalkyl amides corresponding to formula (I): 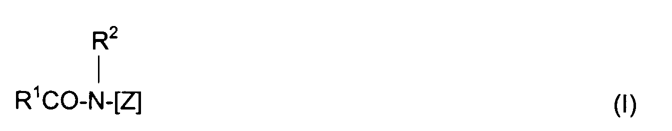 in which R¹CO represents an aliphatic acyl group containing 6 to 22 carbon atoms, R² is an alkyl or hydroxyalkyl group containing 1 to 4 carbon atoms and [Z] is a linear or branched polyhydroxyalkyl group containing 3 to 12 carbon atoms.

3. Nonionic surfactant concentrates as claimed in claims 1 and 2, characterized in that they contain fatty acid-N-alkyl glucamides.

4. Nonionic surfactant concentrates as claimed in claims 1 to 3, characterized in that they contain at least two alcohol polyglycol ethers of which at least one has an HLB value of 2 to 4 and at least one an HLB value of 8 to 12.

5. Nonionic surfactant concentrates as claimed in claims 1 to 4, characterized in that they have an active substance content of 30 to 100% by weight.

6. Nonionic surfactant concentrates as claimed in claims 1 to 5, characterized in that they have a fatty acid-N-alkyl polyhydroxyalkyl amide content of 25 to 90% by weight.

7. Nonionic surfactant concentrates as claimed in claims 1 to 6, characterized in that they have an alcohol polyglycol ether content of 5 to 75% by weight.

8. Nonionic surfactant concentrates as claimed in claims 1 to 7, characterized in that they contain the alcohol polyglycol ethers with HLB values below and above 5 in a ratio by weight of 10:90 to 90:10.

9. A process for the production of nonionic surfactant concentrates in which at least two alcohol polyglycol ethers of which at least one has an HLB value below 5 and at least one an HLB value above 5 are added to fatty acid-N-alkyl polyhydroxyalkyl amides.

10. The use of mixtures of at least two alcohol polyglycol ethers of which at least one has an HLB value below 5 and at least one an HLB value above 5 as crystallization inhibitors for the production of the nonionic surfactant concentrates claimed in claim 1.

## Revendications

1. Concentrés tensioactifs non ioniques contenant
a) des N-alkylpolyhydroxyalkylamides d'acides gras et
b) au moins deux alcoolpolyglycoléthers, dont au moins un présente une valeur d'EHL inférieure à 5 et au moins un une valeur d'EHL supérieure à 5.

2. Concentrés tensioactifs non ioniques selon la revendication 1,
caractérisés en ce qu'ils
contiennent des N-alkylpolyhydroxyalkylamides d'acides gras de formule **(I)** 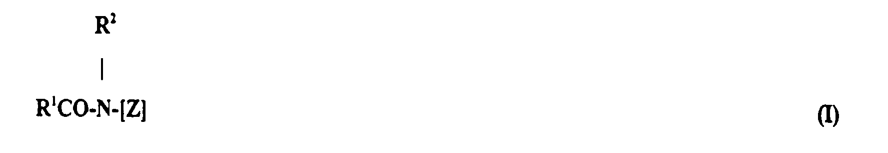 dans laquelle R¹CO représente un radical acyle aliphatique ayant 6 à 22 atomes de carbone, R² représente un radical alkyle ou hydroxyalkyle ayant 1 à 4 atomes de carbone et [Z] représente un radical polyhydroxyalkyle linéaire ou ramifié ayant de 3 à 12 atomes de carbone.

3. Concentrés tensioactifs selon les revendications 1 et 2,
caractérisé en ce qu'
ils contiennent des N-alkylglucamides d'acides gras.

4. Concentrés tensioactifs non ioniques selon les revendications 1 à 3,
caractérisés en ce qu'
ils contiennent au moins deux alcoolpolyglycoléthers dont au moins un présente une valeur d'EHL comprise entre 2 et 4 et au moins un présente une valeur d'EHL comprise entre 8 et 12.

5. Concentrés tensioactifs non ioniques selon les revendications 1 à 4,
caractérisés en ce qu'
ils présentent une teneur en matière active comprise entre 30 et 100 % en poids.

6. Concentrés tensioactifs non ioniques selon les revendications 1 à 5,
caractérisés en ce qu'
ils contiennent des N-alkylamides d'acides gras dans un intervalle de 25 à 90 % en poids.

7. Concentrés tensioactifs non ioniques selon les revendications 1 à 6,
caractérisés en ce qu'
ils présentent une teneur en alcoolpolyglycoléthers comprise entre 5 et 75 % en poids.

8. Concentrés tensioactifs non ioniques selon les revendications 1 à 7,
caractérisés en ce qu'
ils contiennent les alcoolpolyglycoléthers ayant une valeur d'EHL située en-dessous ou selon les cas en-dessous de 5 dans un rapport pondéral de 10:90 à 90:10.

9. Procédé de fabrication de concentrés tensioactifs non ioniques, dans lequel on ajoute à des N-alkylpolyhydroxyalkylamides d'acides gras au moins deux alcoolpolyglycoléthers dont au moins présente une valeur d'EHL inférieure à 5 et au moins un présente une valeur d'EHL supérieure à 5.

10. Utilisation de mélanges d'au moins deux alcoolpolyglycoléthers dont au moins un présente une valeur d'EHL inférieure à 5 et au moins un présente une valeur d'EHL supérieure à 5, comme inhibiteurs de cristallisation pour la fabrication de concentrés tensioactifs non ioniques selon la revendication 1.
